# EUROPEAN PATENT APPLICATION

(11) **EP 4 006 910 A1**
(43) Date of publication of application: **01.06.2022**
(21) Application number: 20210781.9
(22) Date of filing: 30.11.2020
(51) Int. Cl.: G16H 10/60, G06K 19/06, G06Q 10/08, G16H 20/10, G16H 20/13, G16H 40/20, G16H 70/20, G16H 70/40

(54) **DATA EVALUTATOR, METHOD FOR DATA AQCUISITION AND COMPUTER PROGRAM PRODUCT**

(71) Applicant: Aesculap AG, 78532 Tuttlingen (DE)
(72) Inventor: KOCH, Jan, 78532 Tuttlingen (DE); VITR, Mirco, 52074 Aachen (DE)
(74) Representative: Winter, Brandl - Partnerschaft mbB

(57) **Abstract**

A method for data acquisition of consumption of an item or medical device in an activity from barcodes in an image containing one or more barcodes is disclosed. It comprises acquiring barcode information, comprising device identifier information, production identifier information, and barcode type information, retrieving additional item information from a database, determining whether a predetermined required set of information for the item is present in the barcodes, and if yes, the minimal set of barcodes, that contain the predetermined required set of information for the item, according to predetermined rules, transmitting marking information, and storing the predetermined required set of information. Devices, Methods and a computer program product are also described.

## Description

### TECHNICAL FIELD

The present invention refers to processing of coded information on items. An exemplary embodiment is the coded information usually detected by a barcode scanner. In particular the invention can be used to process the information scanned from items with more than one barcode and to determine the information contained therein. Preferably the scanner can determine relevant information in dependence of the intended use. One field of usage is the processing and documentation of medical devices. The invention can be employed for other usages as well, for example in logistics of any items.

### BACKGROUND OF THE INVENTION

The following description of the prior art serves to explain the invention and in particular the advantages of the invention. It is noted that any discussion of the prior art is neither explicit nor implicit concession of the prior art being widely known or common knowledge in the field of the invention.

Keeping track and documenting the medical devices that are used in connection with a patient or a medical procedure for a patient is essential, in order to be able to trace back the devices, wherein a device can be any type of medical product as well, in case of an infection or device recall. It is necessary to be able to determine which specific items, including their lot or serial number information, have been employed, e.g. implanted or used, in context with a patient. A lot number can also be called batch number sometimes.

Currently manual documentation is common, which includes removing detachable stickers from devices. Frequently these stickers are collected by the personnel in one way or the other. For example, it is known that the stickers are attached to the scrubs being worn during the procedure.

Later, at a workstation which is used for documentation purposes, the stickers are removed and attached onto forms or paper, which can then in turn be archived and/or digitalized.

Disadvantageously, some devices do not have stickers. In this case the information, for example a lot, can be printed on the item, the items packaging, or the packaging of a set of items. These items are prone to be forgotten and thus missing in the documentation later-on.

In the systems currently used in a hospital, for example hospital information system (HIS), hospital management software or hospital management system (HMS), laboratory information system (LIS), policy and procedure management system, radiology information system (RIS), picture archiving and communication system (PACS), enterprise resource planning (ERP) system, or the like, the usage of medical items for a patient can be digitally documented. Often a barcode scanner is employed to collect the information from the collected stickers.

Two primary standards have prevailed for medical devices, GS1 (global standards 1), e.g. GTIN (global trade item number), and HIBC (health industry barcode) systems. For certain devices also the Pharmaceutical central number (PZN, for German "Pharmazentralnummer") is used. In order to relate the correct item to the barcode that is scanned, a database has to be maintained containing master data of all items possibly used. Therefore, the information system used needs a materials management block or an interface between the information system and a material management software is necessary.

As an exemplary research has shown, about 27 percent of the data sets of implants that were scanned in a hospital were not maintained correctly, i.e. the GTIN was missing. Consequently, the barcodes could be scanned, but not processed correctly. In such cases, the user is merely shown an error message, with no possibility to remedy the data other than to enter the missing data manually.

This is in particular detrimental, since - at least in some countries - implants, like knee and hip replacement implants need to be documented on a legal basis. With the manual entry of implant data after scanning, human error can contribute to a poor quality of the data in the system. Erroneous data can also lead to claim for damages, therefore having a correct database of all items used is very desirable.

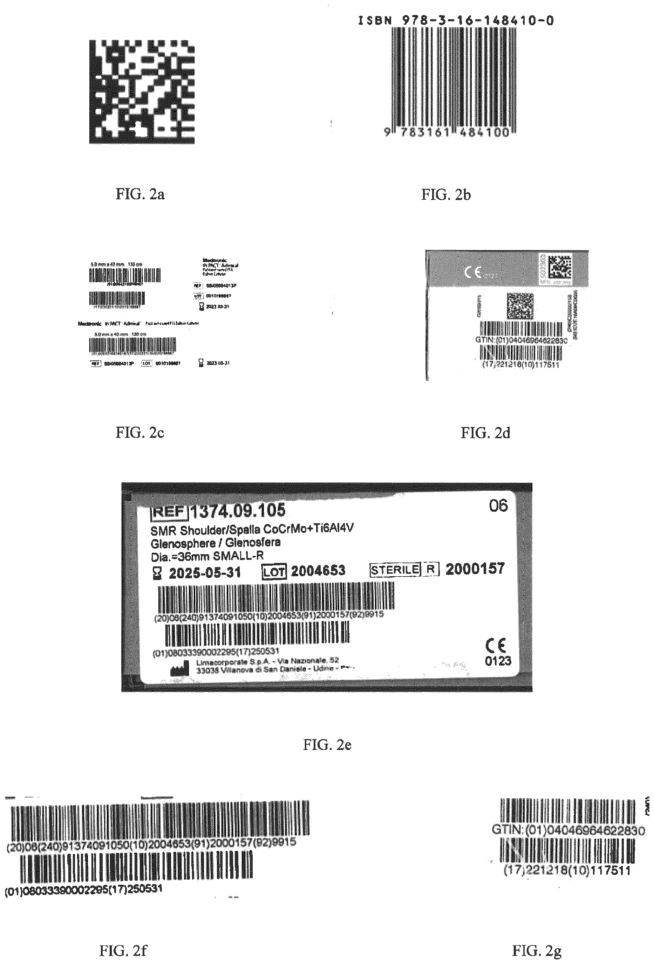

Further, some devices or lots have more than one barcode on them. The manufacturers of medical devices have a certain liberty on how to code the relevant information on their devices. In particular, the GS1 standard can be interpreted differently, leading to inconsistent presentation of information on the devices from different manufacturers. It would be desirable that all relevant information is present in one scannable element, preferably a data matrix code or a similar code. A data matrix is 2D code, as shown in Fig. 2a, which can require less surface than a conventional barcode. Some data matrix principles are describing in DE4107020A1. With a data matrix of the size 48 rows and 48 columns, which is a common size of data matrixes, 348 numerical or 259 alphanumerical characters can be indicated. Depending on the printed resolution, a 48 x 48 data matrix would be of the size 10 x 10 mm up to 20 x 20 mm. According to the defining standard ECC 200 currently used, the largest data matrix for common use is 144 x 144, which can hold up to 3116 numerical or 2355 alphanumerical characters.

In comparison to that, with a conventional barcode on a book, the books international standard book number (ISBN), which is a 13-digit number, is indicated. Such a barcode is shown in Fig. 2b.

Relevant information for documentation of medical devices include the GTIN, an expiration date, and a lot or serial number. However, in reality, this information, and possibly further information, which is irrelevant for the documentation, is often place on the medical devices, as can be seen in Figs. 2c to 2g, showing real-world examples. In Fig. 2c the lowest of three barcodes would contain all relevant information. In Fig. 2d the lower of two data matrixes would be the correct one to be scanned for all relevant information. Users are often confused and overwhelmed, resulting in unnecessary scanning procedures and an increased probability for errors leading to incorrect data sets. Other devices do not have a single code item that contains all relevant information, but several barcodes or data matrixes. Scanning with prior art techniques results in an incomplete documentation in these cases. Fig. 2e shows an example, where the lower barcode contains the GTIN (i.e. value after indicator field "(01)") and the expiration data (i.e. value after indicator field "(17)") while the upper barcode contains the lot number (i.e. value after indicator field "(10)").

The current procedure required that the lot number must be scanned first, in order to indicate to the information system, that further information will follow in addition to the GTIN. If the GTIN is scanned first, the lot number will be joined with the following GTIN. The documentation would be erroneous, and there is no warning or other indication to the user.

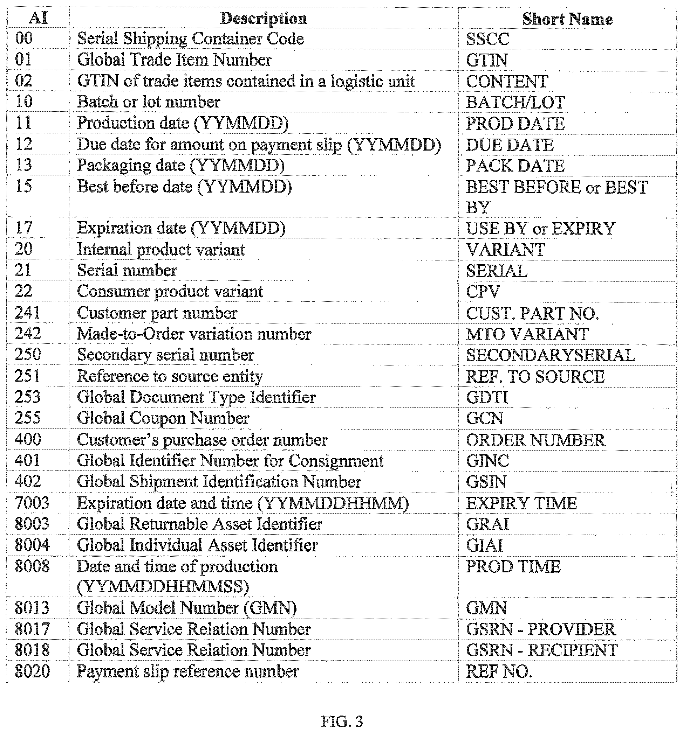

Many barcode scanners are designed to detect data matrix codes by not employing a single line target ray highlighting the barcode, but rather to scan in a rectangular area. If more than one code is within this area, the scanner has to determine which code to scan and evaluate first. Experienced personnel is known to cover the codes that must not be scanned or not be scanned at first, such that the scanning efficiency is improved. That means, the user covers the code containing the GTIN and scan the lot number first. However, the indicator fields of the GS1 standard are not commonly known, and different fields can be used to indicate similar information. The GS1 standard known more than 500 different so-called application identifiers. Fig. 3 shows an excerpt of GS1 application identifiers. Also, some users are mistaken to always scan the longer code first or to scan the codes in vertical order, while in reality, neither the length nor the position divulge any of the contents of the codes, instead the user would be required to look for the application identifier "(10)", which is in the upper code in Fig. 2f but in the lower code in Fig. 2g.

The object of the present invention is to overcome the disadvantages of the prior art, to improve the current situation with the prior art, and/or to at least offer a useful alternative to the existing prior art.

### BRIEF SUMMARY OF THE INVENTION

The present invention solves the aforementioned objection with the subject-matter of the independent claims. Advantageous further developments and refinements result from the dependent claims.

The present invention provides for a method that can supply correct item information to a data base. In connection with the master data stored therein already, documentation of usage of medical devices and thus patient safety is improved.

As an additional advantage, reordering of devices to refill stock to a predetermined number of items can easily be automated, if the information system is ensured to have correct usage information in real time. Therefore, the information of used stock items is transmitted, immediately or deferred, e.g. daily or weekly, to the producer and/or vendor of the respective items. Supported by premade arrangements for ordering and payment, the items can then automatically be order, shipped and delivered, such that a hospital always has the desired amount of each medical device item in stock.

Embodiments of the present invention refer to a method for data acquisition of consumption of an item of medical material in an activity from barcodes in an image containing one or more barcodes. The method comprises a step of acquiring barcode information of at least one barcode. The barcode information can comprise device identifier information, for example a GTIN, and/or a manufacturer identifier, e.g. a labeler identification code (LIC), and/or a manufacturer's number, e.g. a product or catalog number (PCN). Sometimes a REF number is used, which represents a producer's reference number. The barcode information can comprise production identifier information, line a lot number, a serial number, and/or an expiration date. The barcode information can further comprise barcode type information, indicating which barcode was detected and decoded, e.g. EAN13, data-matrix, Code-128, or Code 39. In case the REF is used, the producer must be scanned or alternatively input.

The method then proceeds to retrieving additional item information from a database on the basis of the acquired information, and to determining on the basis of the acquired information and the retrieved information, whether a predetermined required set of information for the item is present in the barcodes.

If this determination results in a positive result, the minimal set of barcodes, that contain the predetermined required set of information for the item is then according to predetermined rules determined.

The method then proceeds to transmitting marking information based on the result of the determining for displaying the image and providing marking for the barcodes contained in the image. And finally, the method proceeds to storing the predetermined required set of information for the item in association with the activity in a memory.

According to one embodiment, the marking can be performed by one of an overlay color, a shape around the barcode, text, a number, and/or a symbol at or near the barcode, and different markings can be used to indicate different conditions. Examples of these conditions include: Barcode(s) which comprise the predetermined required set of information for the item, which can be for example be brushed, i.e. overlaid, with green color. Barcode(s) which do not comprise the full required set of information for the item can be brushed yellow. Invalid barcodes can be brushed orange. Invalid here means that the barcode is not compliant with any recognized standard. Valid barcodes, which cannot be found in the data base can be brushed blue. And valid codes, which hold redundant information, and thus are not part of the minimal set can be brushed white.

Of course, brushing is only one example of marking, and other display elements can be used and the colors can be changed.

According to one embodiment, it can be determined whether the item is associated with the activity and/or context. An example of a current activity is the operation that is currently performed on the patient.

According to one embodiment, a different marking can be used for one or more barcodes which indicate that the item is not associated with the activity.

According to one embodiment the predetermined required set of information for the item comprises one or more of a unique item number, e.g. a GTIN, a manufacturer identifier, e.g. a LIC, a manufacturer's item number, e.g. a PCN, a serial number, a lot number, and/or an expiration date.

According to one embodiment the additional item information from the database comprises the predetermined required set of information for the item. Additionally or alternatively, the predetermined required set of information for the item comprises one or more of a serial number, a lot number, and/or an expiration date.

According to one embodiment, the method can further comprise one or more of acquiring the image; detecting the barcodes in the image; decoding the detected barcodes on the basis of barcode coding tables; interpreting the decoded barcodes on the basis of interpretation tables; and/or displaying the acquired image together with the markings.

According to one embodiment, the method further comprises prompting a user and receiving additional input from the user, if at least one invalid barcode is determined, the predetermined required set of information for the item is not determined to be present in the barcodes, and/or if the method has terminated successfully.

According to one embodiment the method further comprises displaying interaction elements, preferably based on the barcode information and/or additional item information. Thereby, a user manual for an item can be linked or displayed. Such a link can be included in the barcode or in the database.

According to one embodiment the method is repeatedly performed processing a plurality of images, preferably after lapse of a predetermined time and/or a user confirmation.

Another embodiment refers to a data evaluator. It is clear that the above aspects also represent a description of the corresponding aspects of a data evaluator, where a device or a part thereof corresponds to a method step or a feature of a method step.

Analogously, aspects described in the context of a method step in this document also represent a description of a corresponding apparatus or part of an apparatus or item or feature of a corresponding apparatus.

Some or all of the method steps may be executed by (or using) a hardware apparatus, like for example, a microprocessor, a programmable computer or an electronic circuit.

Another embodiment refers to a computer program product including a program for a processing device according to above detailed aspects.

### BRIEF DESCRIPTION OF THE DRAWINGS

The above summary, further objectives, features, advantages, and embodiments according to the present invention will subsequently be described taking reference to the enclosed figures in which:

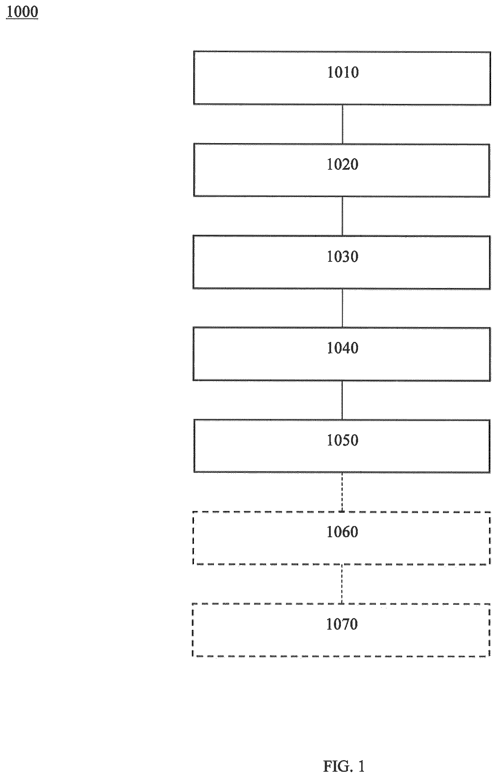

- Fig. 1: shows a flow chart of a method of the invention according to one embodiment,
- Figs. 2a to 2g: show real-world examples of codes on devices,
- Fig. 3: is a list of GS1 standard application identifiers,
- Figs. 4a to 4x: show different product coding scheme, and
- Fig. 5: shows an exemplary barcode decoding table,

In the figures, similar reference signs denote similar elements and features.

### DETAILED DESCRIPTION OF THE INVENTION

In the following, examples of the present disclosure will be described in detail using the accompanying descriptions. In the following description, many details are described in order to provide a more thorough explanation of examples of the disclosure. However, it will be apparent to those skilled in the art that other examples can be implemented without these specific details. Features of the different examples described can be combined with one another, unless features of a corresponding combination are mutually exclusive or such a combination is expressly excluded.

It should be pointed out that the same or similar elements or elements that have the same functionality can be provided with the same or similar reference symbols or are designated identically, with a repeated description of elements that are provided with the same or similar reference symbols or the same are typically omitted. Descriptions of elements that have the same or similar reference symbols or are labeled the same are interchangeable.

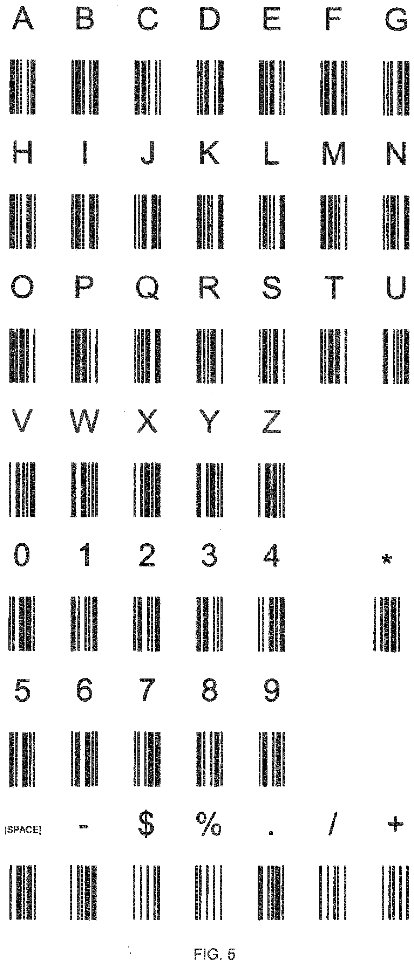

Present scanning apparatuses are able to acquire an image of an item that is placed before the scanner, and to detect and decode the barcodes thereon. Each barcode is decoded in dependence of its coding tables. For a barcode the individual coded characters are coded in the geometry of the bars and spaces of the barcode. As an example, a Code 39 character consists of 5 bars and 4 spaces with 3 of them wide (for a binary value of 1) and 6 of them narrow (for a binary value of 0) for a total of nine elements. It encodes 43 different upper case alphabetic, numeric, graphic characters plus a space and has the capability to encode all 128 ASCII characters. An example of a decoding table, in this case for Code 39, can be seen in Fig. 5. The decoding portion processed the detected code and returns a sequence of decoded characters.

The decoded information can then be interpreted by using existing standard tables. The interpretation tables can for example be a GS1 standard application identifier list. In this case, a decoded character sequence "(01)458996542" can be interpreted to "Global Trade Item Number 458996542" based on the entry "(01) - Global Trade Item Number" in the GS1 standard table. By decoding, a set of decoded information is obtained for each code.

From a data base data can be retrieved, configuring what a complete set of information is required to consist of. For example, for a medical device, a data base can comprise that an expiration date is required, or with a vaccine, a transportation duration time must not be exceeded. Such constraints can be added from a database. Also, changed requirements can be loaded in such manner, for example, while in transit, a recall information is added to the database, and thus the scanned item will be marked "expired" or "recall", thereby its usage can be avoided.

Optionally, the display can indicate to the user the code or codes that are eventually used, since they contain all the necessary information. This can be achieved, for example with a colored or monochrome overlay over the code(s), a colored or monochrome frame around the code(s), a symbol, e.g. a dot, star, or arrow next to the code(s) or any other visible indication.

The remaining information can then be enriched with additional information selected from the master data of the database. Examples are manufacturer information, hospital item number, or item description.

However, devices are not always marked in such a way that information is contained in a single barcode or data matrix. The GS1 standard allows information to be distributed across multiple barcodes. When using a conventional barcode scanner, the user has to decide which code is to be scanned first or leave the decision to the barcode scanner. However, in that cases, errors in the documentation are likely, as detailed above.

With the present invention, it is possible to read several codes at the same time. If a complete set of information cannot be obtained from a single code, two or more codes are combined in the manner described above.

It is known that the scope of a set of information which is considered to be complete, depends on the type of item to be scanned, and can also depend on the current context and/or activity. For an implant for example a complete set could be constituted by GTIN + expiry date + lot number or GTIN + expiry date + serial number.

In order to arrive at a complete set, incomplete set retrieved by scanning can be completed by manual entries, as follows:
Scanned: GTIN only. Manual entry: expiry date + lot or serial number.
Scanned: GTIN + expiry date. Manual entry: lot or serial number.
Scanned: GTIN + lot. Manual entry: expiry date.
Scanned: GTIN + serial number. Manual entry: expiry date.

The invention aims at making the documentation as easy as possible for the user. Therefore, the display could work with different colored brushes, e.g. overlays, so that users are made aware of and recognize different coding standards over time. The master data can be used to define how the article should be coded, which codes are expected and which information is to be documented. A brush is here a colored overlay indicating a marking of a certain area, like a highlighter brush on paper.

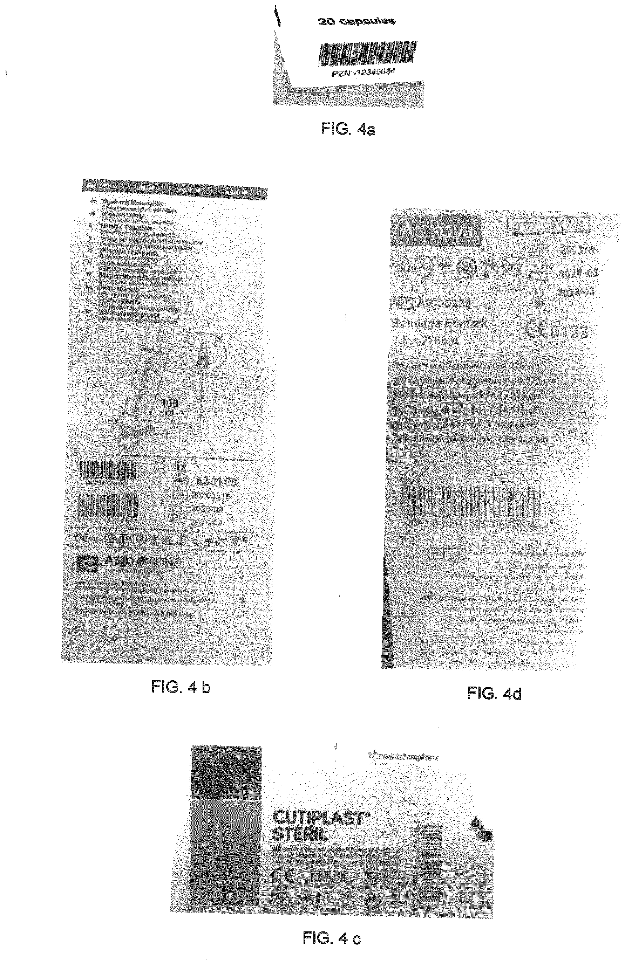

In the following some examples for medical device coding is detailed with reference to Figs. 4a to 4x. It is noted that the same principles are applicable to devices in other fields as well.

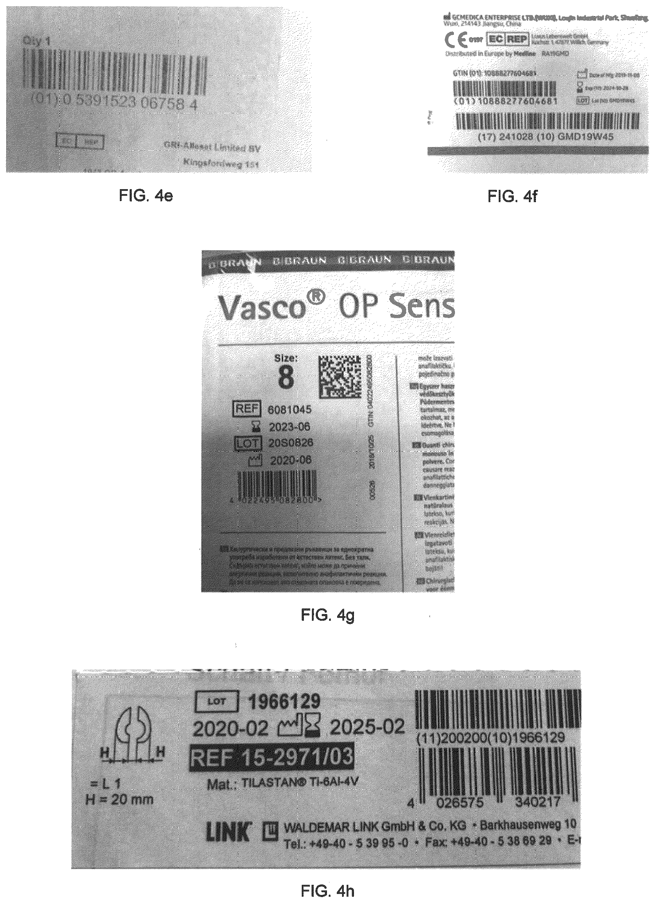

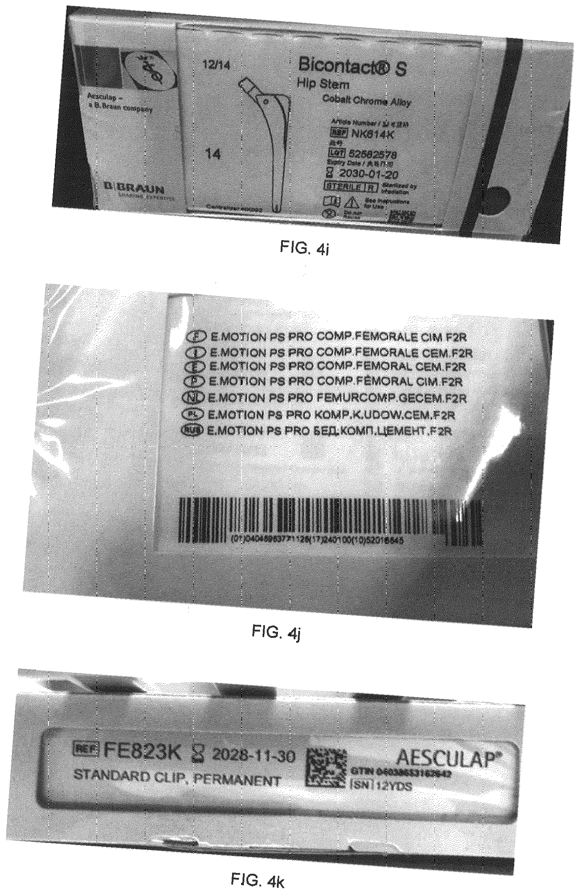

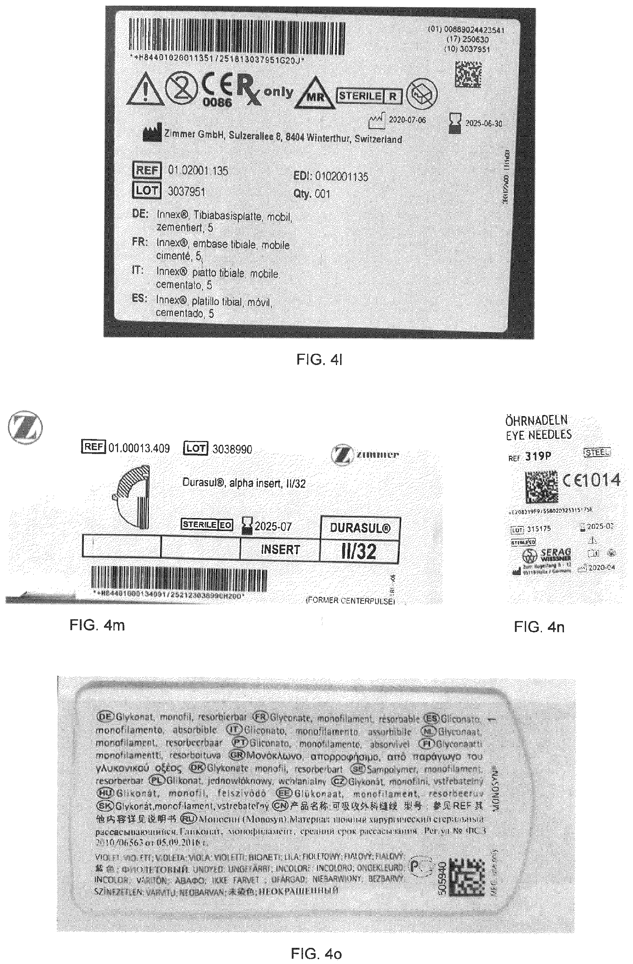

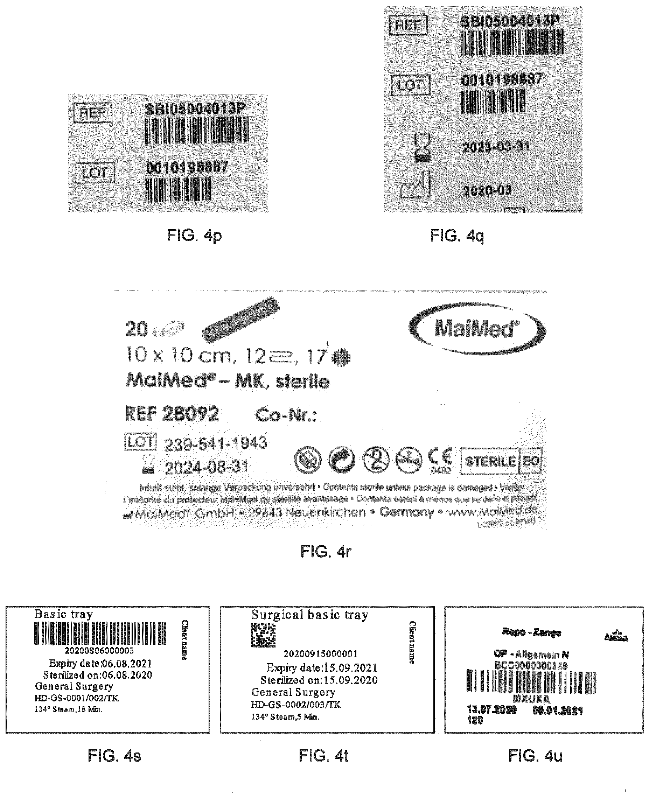

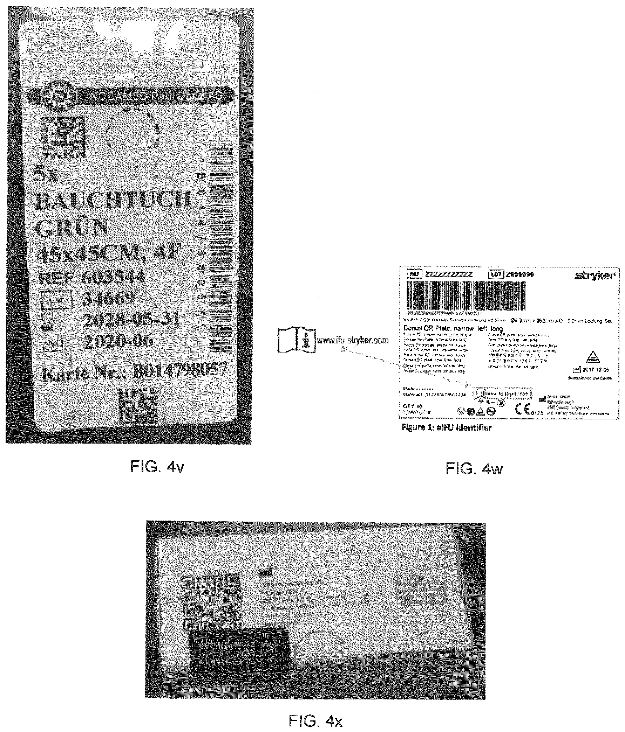

As one example for medical devices, implants are detailed in the following. It is common for implants to bear coded information relating to the GTIN/LIC+PNC, expiry date, and lot or serial number. Known coding schemes for medical devices include:
- Pharmaceutical central number (PZN, for German "Pharmazentralnummer") (shown in Fig. 4a)
- European Article Number (EAN13 + PZN) (shown in Fig. 4b)
- EAN13 (shown in Fig. 4c)
- GS1 (01) (shown in Fig. 4d)
- GS1 (01) + GS1 (17 / 10) (shown in Fig. 4e and 4f)
- GS1 (01 / 17 / 10) + EAN 13 (shown in Fig. 4g)
- GS1 (* / 10) + EAN 13 (shown in Fig. 4h)
- GS1 (01 /17 /10) (shown in Fig. 4i and 4j)
- GS1 (01 / 17 / 21) (shown in Fig. 4k)
- GS1 + HIBC (shown in Fig. 4l)
- HIBC (shown in Fig. 4m and 4n)
- non standardized codes (shown in Fig. 4o and 4p)
- OCR in combination with barcodes (shown in Fig. 4q)
- OCR only (shown in Fig. 4r)
- sterile goods (shown in Fig. 4s, 4t, and 4u)
- Card number (shown in Fig. 4v)
- Documentation or re-sterilized implants
- eIFU, electronic instructions for use (shown in Fig. 4w)
- QR-Code (shown in Fig. 4x)
- validating the expiry date.

In the above, OCR, i.e. optical character recognition, comprises recognizing text comprised by individual letters, but also recognition of symbols, i.e. graphical symbols, for example the designation "LOT" in Fig. 4p or 4q or the hourglass symbol indicating an expiry date in Fig. 4q. Thus, this can also refer to image recognition and/or include machine learning to learn the meaning of commonly used symbols.

PZN (Fig. 4a): In Germany, drugs bear a pharmaceutical central number (PZN), in the European Union, a pharmacy product number, PPN, is used. The PZN or PPN standard has no priority for documenting and re-ordering of implants. However, in order to correctly track and re-fill hospital supplies, these codes need to be detected and interpreted correctly. The PZN should be marked differently, i.e. different color, shape, and/or symbol, from the GS1 code(s).

EAN13 + PZN (Fig. 4b): Since the PZN does not correspond to EAN13, both codes must be interpreted to identify the article. When creating new master data, the user should be asked whether the article contains other machine-readable codes. The joining of PZN and EAN13 is important so that the data can be correctly transmitted to materials management when ordering. An article that has been completely created in the master data should be identified via PZN or EAN13. The PZN should be marked differently, i.e. different color, shape, and/or symbol, from the GS1 code(s).

EAN13 (Fig. 4c): For articles with an EAN13, the manual entry of an expiry date and lot or serial number is expected. When scanning and thereby creating new master data, the user should be asked whether the item contains other machine-readable codes and whether the expiry date and lot or serial number should be documented. Generally, manual input should be possible, but only required if it is necessary for the respective device. For example, manually recording the lot number for plasters is not justified for the device. Also, documenting an implant without a lot number would not comply with the documentation requirement. This is also detailed below with context to other GS1 coding schemes.

GS1 with identifier (01) (Fig. 4d): There are devices with a barcode, that contain a GTIN with the identifier (01), but no other codes. While an EAN13 can be processed immediately, prior art scanners wait after scanning a GS1 (01) code for other codes to be scanned, until the user interacts, e.g. pressing a button "proceed", The master data in this case can indicate that no further codes are to be expected for this device. Manual entry of lot information can also be depending on the master data.

To conclude, the embodiments described herein can optionally be supplemented by any of the important points or aspects described here. However, it is noted that the important points and aspects described here can either be used individually or in combination and can be introduced into any of the embodiments described herein, both individually and in combination.

Although some aspects have been described in the context of an apparatus, it is clear that these aspects also represent a description of the corresponding method, where a device or a part thereof corresponds to a method step or a feature of a method step. Analogously, aspects described in the context of a method step also represent a description of a corresponding apparatus or part of an apparatus or item or feature of a corresponding apparatus. Some or all of the method steps may be executed by (or using) a hardware apparatus, like for example, a microprocessor, a programmable computer or an electronic circuit. In some embodiments, one or more of the most important method steps may be executed by such an apparatus.

Depending on certain implementation requirements, embodiments of the invention can be implemented in hardware or in software. The implementation can be performed using a digital storage medium, for example a floppy disk, a DVD, a Blu-Ray, a CD, a ROM, a PROM, an EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may for example be stored on a machine-readable carrier.

Other embodiments comprise the computer program for performing one of the methods described herein, stored on a machine-readable carrier.

In other words, an embodiment of the inventive method is, therefore, a computer program having a program code for performing one of the methods described herein, when the computer program runs on a computer.

A further embodiment of the inventive methods is, therefore, a data carrier (or a digital storage medium, or a computer-readable medium) comprising, recorded thereon, the computer program for performing one of the methods described herein. The data carrier, the digital storage medium or the recorded medium are typically tangible and/or non-transitionary.

A further embodiment of the inventive method is, therefore, a data stream or a sequence of signals representing the computer program for performing one of the methods described herein. The data stream or the sequence of signals may for example be configured to be transferred via a data communication connection, for example via the Internet.

A further embodiment comprises a processing means, for example a computer, or a programmable logic device, configured to or adapted to perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

The apparatus described herein may be implemented using a hardware apparatus, or using a computer, or using a combination of a hardware apparatus and a computer. The apparatus described herein, or any components of the apparatus described herein, may be implemented at least partially in hardware and/or in software.

The methods described herein may be performed using a hardware apparatus, or using a computer, or using a combination of a hardware apparatus and a computer. The methods described herein, or any parts of the methods described herein, may be performed at least partially by hardware and/or by software.

The above described embodiments are merely illustrative for the principles of the present invention. It is understood that modifications and variations of the arrangements and the details described herein will be apparent to others skilled in the art. It is the intent, therefore, to be limited only by the scope of the impending patent claims and not by the specific details presented by way of description and explanation of the embodiments herein.

## Claims

1. Method (1000) for data acquisition of consumption of an item of medical material in an activity from barcodes in an image containing one or more barcodes, comprising
acquiring (1010) barcode information of at least one barcode, wherein each barcode information comprises device identifier information, production identifier information, and barcode type information,
retrieving (1020) additional item information from a database on the basis of the device identifier information, production identifier information, and barcode type information;
determining (1030) on the basis of the device identifier information, the production identifier information, the barcode type information, and the additional item information, whether a predetermined required set of information for the item is present in the barcodes, and if yes, the minimal set of barcodes, that contain the predetermined required set of information for the item, according to predetermined rules;
transmitting (1040) marking information based on the result of the determining for displaying the image and providing marking for the barcodes contained in the image; and
storing (1050) the predetermined required set of information for the item in association with the activity in a memory.

2. Method according to claim 1, wherein a marking information can refer to one of an overlay color, a shape around the barcode, text, a number, and/or a symbol at or near the barcode,
and/or wherein different markings can be used for
one or more of the barcodes which have been determined to comprise the predetermined required set of information for the item;
one or more of the barcodes, if the predetermined required set of information for the item is not determined to be present in the barcodes;
one or more of the barcodes which have been determined to be invalid;
one or more of the barcodes which have been determined to be valid, but no associated additional item information could be retrieved from the database; and/or
one or more of the barcodes which have been not been used in the minimal set of barcodes.

3. Method according to any one of the preceding claims, wherein the step of determining (1030) further comprises determining on the basis of the device identifier information, the production identifier information, the barcode type information, and the additional item information, whether the item is associated with the activity.

4. Method according to claim 3, wherein a different marking can be used for one or more barcodes which indicate that the item is not associated with the activity.

5. Method according to any one of the preceding claims, wherein the predetermined required set of information for the item comprises one or more of a unique item number, a manufacturer identifier, a manufacturer's item number, a serial number, a lot number, and/or an expiration date.

6. Method according to any one of the preceding claims,
wherein the additional item information comprises the predetermined required set of information for the item; and/or
wherein the predetermined required set of information for the item comprises one or more of a serial number, a lot number, and/or an expiration date.

7. Method according to any one of the preceding claims, further comprising one or more of
acquiring the image;
detecting the barcodes in the image;
decoding the detected barcodes on the basis of barcode coding tables;
interpreting the decoded barcodes on the basis of interpretation tables; and/or
displaying (1060) the acquired image together with the markings.

8. Method according to any one of the preceding claims, further comprising prompting (1070) a user and receiving additional input from the user, if at least one invalid barcode is determined, the predetermined required set of information for the item is not determined to be present in the barcodes, and/or if the method has terminated successfully.

9. Method according to any one of the preceding claims, further comprising or wherein the step of displaying (1060) further comprises displaying interaction elements, preferably based on the barcode information and/or additional item information.

10. Method according to any one of the preceding claims, wherein the method is repeatedly performed processing a plurality of images, preferably after lapse of a predetermined time and/or a user confirmation.

11. Data evaluator for data acquisition of consumption of an item of medical material in an activity from barcodes in an image containing one or more barcodes, comprising
an acquiring portion, configured to acquire barcode information of at least one barcode, wherein each barcode information comprises device identifier information, production identifier information, and barcode type information,
a retrieving portion, configured to retrieve additional item information from a database on the basis of the device identifier information, production identifier information, and barcode type information;
a determining portion, configured to determine on the basis of the device identifier information, the production identifier information, the barcode type information, and the additional item information, whether a predetermined required set of information for the item is present in the barcodes, and if yes, the minimal set of barcodes, that contain the predetermined required set of information for the item, according to predetermined rules;
a transmitting portion, configured to transmit marking information based on the result of the determining for displaying the image and providing marking for the barcodes contained in the image; and
a storing portion, configured to store the predetermined required set of information for the item in association with the activity in a memory.

12. Data evaluator according to claim 11, wherein a marking information can refer to one of an overlay color, a shape around the barcode, text, a number, and/or a symbol at or near the barcode,
and/or wherein different markings can be used for
one or more of the barcodes which have been determined to comprise the predetermined required set of information for the item;
one or more of the barcodes, if the predetermined required set of information for the item is not determined to be present in the barcodes;
one or more of the barcodes which have been determined to be invalid;
one or more of the barcodes which have been determined to be valid, but no associated additional item information could be retrieved from the database; and/or
one or more of the barcodes which have been not been used in the minimal set of barcodes.

13. Data evaluator according to any one of claims 11 to 12, wherein the determining portion is further configured to determine on the basis of the device identifier information, the production identifier information, the barcode type information, and the additional item information, whether the item is associated with the activity.

14. Data evaluator according to according to claim 13, wherein a different marking can be used for one or more barcodes which indicate that the item is not associated with the activity.

15. Data evaluator according to according to any one of claims 11 to 14, wherein the predetermined required set of information for the item comprises one or more of a unique item number, a manufacturer identifier, a manufacturer's item number, a serial number, a lot number, and/or an expiration date.

16. Data evaluator according to according to any one of claims 11 to 15,
wherein the additional item information comprises the predetermined required set of information for the item; and/or
wherein the predetermined required set of information for the item comprises one or more of a serial number, a lot number, and/or an expiration date.

17. Data evaluator according to any one of claims 11 to 16, further comprising one or more of
an acquiring portion, configured to acquire the image;
a detecting portion, configured to detect the barcodes in the image;
a decoding portion, configured to decode the detected barcodes on the basis of barcode coding tables;
an interpreting portion, configured to interpret the decoded barcodes on the basis of interpretation tables; and/or
a display portion, configured to display the acquired image together with the markings.

18. Data evaluator according to according to any one of claims 11 to 17, further comprising an interaction portion, configured to prompt a user and receive additional input from the user, if at least one invalid barcode is determined, the predetermined required set of information for the item is not determined to be present in the barcodes, and/or if data acquisition from an image has terminated successfully.

19. Data evaluator according to according to any one of claims 11 to 18, further comprising a display portion, configured to display interaction elements, preferably based on the barcode information and/or additional item information.

20. Data evaluator according to according to any one of claims 11 to 19, configured to successively process a plurality of images, preferably to proceed to a next image after lapse of a predetermined time and/or a user confirmation.

21. Computer program product including a program for a processing device, comprising software code portions for performing the steps of claims 1 to 10 when the program is run on the processing device.

22. The computer program product according to claim 21, wherein the computer program product comprises a computer-readable medium on which the software code portions are stored, wherein the program is directly loadable into an internal memory of the processing device.
